# EUROPÄISCHE PATENTANMELDUNG

(11) **EP 2 716 621 A1**
(43) Veröffentlichungstag der Anmeldung: **09.04.2014**
(21) Anmeldenummer: 12006925.7
(22) Anmeldetag: 05.10.2012
(51) Int. Cl.: C07C 5/48, C07C 11/04

(54) **Reaktoreinrichtung und Verfahren zur oxidativen Dehydrierung von Alkanen**

(71) Anmelder: Linde Aktiengesellschaft, 80331 Munich (DE)
(72) Erfinder: Hofmann, Karl-Heinz, Dr., 82110 Germering (DE); Meiswinkel, Andreas, Dr., 81479 München (DE); Thaller, Christian, 80687 München (DE); Zander, Hans-Jörg, Dr., 81479 München (DE)
(74) Vertreter: Zahn, Christoph

(57) **Zusammenfassung**

Verfahren zur oxidativen Dehydrierung von Alkanen, insbesondere von Ethan zu Ethylen, bei dem ein Einsatzstoffstrom (E) enthaltend ein Alkan, insbesondere Ethan, sowie Sauerstoff durch zumindest ein einen Katalysator (K) enthaltendes Festbett (10, 11) einer Reaktoreinrichtung (1) geleitet wird, wobei durch oxidatives Dehydrieren des Alkans mit Sauerstoff in Gegenwart des Katalysators (K) ein alkenhaltiger Produktstoffstrom (P) erzeugt wird, und wobei der Katalysator (K) mit einem Inertmaterial (I) verdünnt ist.

## Beschreibung

Die Erfindung betrifft eine ein Verfahren zur oxidativen Dehydrierung von Alkanen zu dem jeweils korrespondierenden Alken sowie eine Reaktoreinrichtung.

Ein bekanntes Verfahren zu Herstellung von Alkenen (Olefinen) wie Ethylen und Propylen ist das Steamcracken von unterschiedlichen Kohlenwasserstoffen. In Frage kommen hier insbesondere Ethan, Propan, Naphtha, Gasöle und andere schwere Einsätze. Ein Nachteil des Steamcracking-Verfahrens besteht darin, dass insbesondere bei schwereren Einsätzen weitere Nebenprodukte anfallen, die vom gewünschten Olefinprodukt abzutrennen sind. Ferner ist das Steamcracken ein energieintensives Verfahren, das bei vergleichsweise hohen Temperaturen im Bereich von ca. 800°C bis 900°C erfolgt. Bei diesem Verfahren werden stets auch Acetylenverbindungen gebildet (z.B. Acetylen, Methylacetylen, Propadien), die eine aufwendige separate Abtrennung durch z.B. Extraktion oder Selektivhydrierung erfordern.

Ein prinzipiell bekanntes Verfahren als Alternative zur Herstellung von C2-C4-Olefinen, insbesondere Ethylen, ist die oxidative Dehydrierung der korrespondierenden Alkane in Gegenwart eines Katalysators.

Ein derartiges Verfahren zur oxidativen Dehydrierung wird z.B. in der WO2010/115108A1 beschrieben, wobei nach der oxidativen Dehydrierung im Produktstoffstrom enthaltener, unreagierter Sauerstoff an einem entsprechenden Katalysator verbrannt wird. Weiterhin wird in der US 6,518,476 B1 als Katalysator für die oxidative Dehydrierung insbesondere ein nicht-stöchiometrischer seltene Erden Oxycarbonat-Katalysator vorgeschlagen. Ferner offenbart die WO2010/115099A1 die Herstellung eines Katalysators für die oxidative Dehydrierung, der neben Mo, V zumindest Nb und/oder Ta, sowie zumindest eines der Elemente Te, Ga, Pd, W, Bi und Al sowie Ni und/oder Sb aufweisen kann. Gemäß EP167109B1 wird als Katalysator bei der Herstellung von Ethylen aus Ethan durch oxidative Dehydrierung u.a. ein MoVNbSbTeMn-Oxid-Katalysator vorgeschlagen. Weiterhin betrifft die US20120016171A1 einen VMoNbTeMe-Oxid-Katalysator, wobei Me aus der Gruppe umfassend Ta, Ti, W, Hf, Zr, Sb stammt. Schließlich schlägt die US20090292153A1 vor, die oxidative Dehydrierung in Gegenwart eines Katalysators sowie von reduzierbaren Metalloxiden vorzunehmen.

Grundsätzlich ist bei der oxidativen Dehydrierung zu beachten, dass erhebliche Wärme durch die exotherm ablaufenden Reaktionen freigesetzt wird. Durch die Anwesenheit von Sauerstoff kann der abgespaltene Wasserstoff zu Wasser umgesetzt werden, als auch die Oxidation zu Kohlenmonoxid bzw. die Totaloxidation zu Kohlendioxid und Wasser aus Ethan bzw. Ethylen erfolgen. Durch die freiwerdende Wärme wird die Katalysatorschüttung bzw. das Festbett sehr heiß und muss gekühlt werden, da ansonsten eine Schädigung des Katalysators erfolgt und/oder die oben erwähnten Nebenreaktionen die Selektivitäten und Ausbeuten des Prozesses nachteilig beeinflussen.

So muss insbesondere bei einem gekühlten isothermen Festbettrohrreaktor der Durchmesser und die Länge des Reaktorrohres, das das Katalysatorfestbett aufnimmt, so dimensioniert werden, dass durch die Außenkühlung des Reaktorrohres (z.B. siedendes Wasser oder Wärmeträgeröl) im Zentrum des Festbettes keine zu hohen Temperaturen auftreten, die zu einem thermischen Durchgehen der Reaktion führen bzw. die Selektivitäten und Ausbeuten verschlechtern. Bei adiabatischer bzw. autothermer Reaktionsführung müssen die Reaktionsbedingungen im Reaktor vorzugsweise so gewählt werden, dass am Reaktoraustritt keine zu hohen Temperaturen entstehen können, die den Katalysator schädigen, zu hohen Nebenproduktanteilen (CO, CO₂) führen bzw. zu einem Durchgehen der Reaktion oder einer Explosion führen bzw. die Selektivitäten und Ausbeuten verschlechtern.

Hiervon ausgehend liegt daher der vorliegenden Erfindung die Aufgabe zugrunde, ein Verfahren und eine Reaktoreinrichtung zur oxidativen Dehydrierung von Alkanen anzugeben, das bzw. die es ermöglicht, der vorgenannten Wärmeentwicklung im Katalysator-Festbett entgegenzuwirken und insbesondere das Risiko einer Explosion bei der oxidativen Dehydrierung zu mindern.

Dieses Problem wird durch ein Verfahren mit den Merkmalen des Anspruchs 1 gelöst. Danach wird bei dem erfindungsgemäßen Verfahren zur oxidativen Dehydrierung von Alkanen, insbesondere von Ethan zu Ethylen, ein Einsatzstoffstrom enthaltend ein Alkan, insbesondere Ethan, sowie Sauerstoff durch zumindest ein einen Katalysator enthaltendes Festbett geleitet, wobei durch oxidatives Dehydrieren des Alkans mit Sauerstoff in Gegenwart des Katalysators ein alkenhaltiger Produktstoffstrom erzeugt wird, und wobei insbesondere der Katalysator zur Beherrschung der Wärmetönung und/oder zur Vermeidung einer Explosion bei der oxidativen Dehydrierung mit einem Inertmaterial verdünnt ist.

Der Grad der Verdünnung wird dabei insbesondere so bemessen, dass die Reaktionstemperatur bei isothermer Reaktionsführung im Reaktorrohr so hoch gehalten werden kann, dass größtmögliche Umsätze und Selektivitäten erreicht werden, ohne dass es zu einem Durchgehen der Reaktion führt. Bei adiabatischer Prozessführung (insbesondere bei mehreren Festbetten) wird der Grad der Verdünnung des aktiven Katalysators und die Länge des ersten Festbetts entlang der Strömungsrichtung der Einsatzstoffstromes bzw. des Produktstoffstromes sowie der Gehalt des verwendeten Feed-Alkans (z.B. Ethan) im Einsatzstoffstrom zum ersten Festbett so bemessen, dass der adiabatische Temperaturanstieg höchste Umsätze bei höchster Selektivität ermöglicht, wobei in einem nachgeschalteten zweiten Festbett auf eine Verdünnung des Katalysators verzichtet werden kann, um den vollständigen Umsatz des verwendeten Alkans (z.B. Ethan) zu ermöglichen (siehe unten).

Besonders bevorzugt wird bei dem erfindungsgemäßen Verfahren als Katalysator ein MoVTeNbOₓ-Katalysator verwendet, wobei jedoch grundsätzlich ein beliebiger Katalysator mit ausreichenden Aktivitäts- und Selektivitätseigenschaften verwendet werden kann, der unter den gegebenen Reaktionsbedingungen stabil ist.

Bei dem erfindungsgemäßen Verfahren weist das mindestens eine Festbett bevorzugt zumindest eine Mehrzahl an jenen Katalysator aufweisenden ersten Partikeln auf, wobei in einer Variante des erfindungsgemäßen Verfahrens jene ersten Partikel auch das Inertmaterial aufweisen.

Gemäß einer weiteren Variante des erfindungsgemäßen Verfahrens ist vorgesehen, dass das mindestens eine Festbett zum Verdünnen des Katalysators eine Mehrzahl an mit den ersten Partikeln regellos vermischten zweiten Partikeln aufweist, die aus dem Inertmaterial gebildet sind. Die ersten und zweiten Partikel sind dabei bevorzugt so vermischt, dass jedes beliebige Teilvolumen des Festbetts, das eine Mehrzahl an ersten und zweiten Partikeln umfasst, im Bereich der besagten Mischung eine im Wesentlichen konstante Dichte bzw. einen konstanten Anteil an ersten und zweiten Partikeln aufweist.

Vorzugsweise weisen die zweiten Partikel eine von den ersten Partikeln, die den aktiver Katalysator aufweisen, abweichende Dimensionierung (z.B. größeres oder kleineres Volumen bzw. größerer oder kleinerer Durchmesser etc.) und/oder eine abweichende Geometrie (z.B. Formgebung) auf, um dadurch den Druckabfall beim Durchströmen der Schüttung bzw. des mindestens einen Festbettes möglichst klein zu halten.

Bei den besagten ersten und zweiten Partikeln handelt es sich vorliegend um makroskopische Partikel, d.h., nicht um einzelne atomare oder molekulare Teilchen. Vorzugsweise weisen diese Partikel eine Korngröße auf, die größer oder gleich einem Mikrometer ist, bevorzugt größer oder gleich 10 Mikrometern, bevorzugt größer oder gleich 100 Mikrometern, bevorzugt größer oder gleich 1 mm. Die Partikel bilden insbesondere Schüttfüllkörper.

Bevorzugt weist bei dem erfindungsgemäßen Verfahren das Inertmaterial einen oder mehrere der folgenden Stoffe auf: Aluminiumoxid, Siliciumdioxid, Siliciumcarbid, Quartz oder Keramik. Idealerweise verfügt das Inertmaterial über gute Wärmeübertragungseigenschaften.

Gemäß einer Variante des erfindungsgemäßen Verfahrens ist vorgesehen, dass die oxidative Dehydrierung des Alkans isotherm erfolgt, wobei das mindestens eine Festbett bevorzugt mittels einer Flüssigkeit gekühlt wird, bei der es sich insbesondere um Wasser oder einen Kohlenwasserstoff oder ein Gemisch der vorgenannten Stoffe handeln kann. Bei einem Kohlenwasserstoff kann es sich dabei um einen nichtfunktionalisierten Kohlenwasserstoff oder um einen funktionalisierten Kohlenwasserstoff (z.B. Alkohol) handeln. Die besagten Flüssigkeiten können beim Kühlen sieden. Weiterhin kann die Kühlung auch mittels eines Wärmeträgeröls oder mittels einer Salzschmelze vorgenommen werden.

Alternativ oder ergänzend (siehe oben) kann die oxidative Dehydrierung auch adiabatisch erfolgen, wobei insbesondere der Einsatzstoffstrom nacheinander durch eine Mehrzahl an Festbetten gegeben wird. Hierbei wird der Einsatzstoffstrom bzw. Produktstoffstrom zwischen je zwei benachbarten Festbetten vorzugsweise zwischengekühlt, z.B. mittels eines Wärmeübertragers und/oder durch Einspritzen von Wasser. Weiterhin wird dem Einsatzstoffstrom vor dem Eintritt in das nächste Festbett bevorzugt jeweils Sauerstoff, Stickstoff und/oder Luft hinzugegeben, wobei Stickstoff in der zugegeben Luft ab- oder angereichert sein kann.

Vorzugsweise beträgt der Anteil des Inertmaterials an dem mindestens einen Festbett bei isothermer Reaktionsführung 30 Vol.-% bis 90Vol.-%, bevorzugt 50Vol.-% bis 80Vol.-%, besonders bevorzugt 60 Vol.-% bis 80Vol.-%. Bei adiabatischer Reaktionsführung beträgt der Anteil des Inertmaterials an dem mindestens einen Festbett vorzugsweise 30 Vol.% bis 90 Vol.%, bevorzugt 50 Vol.-% bis 85 Vol.-%, besonders bevorzugt 60 Vol.% bis 80 Vol.%. Der restliche Anteil wird jeweils durch den (aktiven) Katalysator gebildet.

Gemäß einer Variante des erfindungsgemäßen Verfahrens ist vorgesehen, dass der Einsatzstoffstrom bei einem Druck von 0,5 bar bis 35 bar, bevorzugt 1 bar bis 15 bar, besonders bevorzugt 1 bar bis 10 bar durch das mindestens eine Festbett geleitet wird.

Weiterhin wird der Einsatzstoffstrom bevorzugt bei einer Temperatur von 250°C bis 650°C, bevorzugt 280°C bis 550°C, besonders bevorzugt 350°C bis 480°C durch das mindestens eine Festbett geleitet.

In einer Variante des erfindungsgemäßen Verfahrens ist weiterhin vorgesehen, dass der Einsatzstoffstrom mit einem Verdünnungsmittel verdünnt wird, dass inert ist oder zumindest eine inerte Komponente aufweist.

Das Verdünnungsmittel wird bevorzugt eingesetzt, um die Wärmetönung der Reaktion zu beherrschen. Insbesondere soll die Reaktion außerhalb eines Explosionsbereiches erfolgen.

Vorzugsweise wird als Verdünnungsmittel Wasserdampf, Stickstoff und/oder Luft verwendet, wobei insbesondere Stickstoff und/oder Sauerstoff in der besagten Luft an- oder abgereichert sein kann und in der Luft enthaltener Sauerstoff mit Vorteil als Oxidationsmittel verwendet wird. Die vorgenannten Verdünnungsmittel können auch in beliebiger Kombination eingesetzt werden.

Beim Anfahren der Anlage, d.h., beim Einleiten der oxidativen Dehydrierung, wird bevorzugt Wasserdampf und/oder Stickstoff in die Reaktoreinrichtung gegeben und sodann werden die Reaktanden hinzugefügt, wobei als Oxidationsmittel Sauerstoff oder Luft hinzugegeben wird. In der Luft kann Sauerstoff und/oder Stickstoff an- oder abgereichert sein. Das beim Anfahren hinzugegebene Verdünnungsmittel kann nach dem Anfahren weiterhin in die Reaktoreinrichtung gegeben werden oder zumindest teilweise oder vollständig durch ein anderes Verdünnungsmittel (Wasserdampf, Stickstoff und/oder Luft) ersetzt werden.

Die Verwendung von Dampf als Verdünnungsmittel ist insbesondere von Vorteil, da der Dampf zum einen leicht und günstig erzeugt werden kann, und zum anderen auf einfache Weise durch Kondensation abgeschieden werden kann. Des Weiteren kann dabei zugleich eine Reinigung bzw. ein Auswaschen von Verunreinigungen, wie z.B. Säuren und anderen wasserlöslichen Verbindungen, z.B. Carbonylverbindungen, erfolgen. Die Dampfverdünnung bedingt darüberhinaus lediglich geringe Investitionskosten und kann sogar aktivitäts- und/oder selektivitätsfördernd sein.

Bei Verwendung eines Inertmediums (insbesondere Stickstoff) zur Verdünnung in der Reaktoreinrichtung können mit Vorteil Explosionsgrenzen eingehalten werden, d.h., explosionsfähigen Atmosphären vermieden werden, und zwar sowohl in der Reaktoreinrichtung als auch im Trennteil stromab der Reaktoreinrichtung. Dabei kann der Betrieb der Anlage außerhalb von Explosionsbereichen auch bei erhöhtem Druck erfolgen. Weiterhin kann Stickstoff durch Einspeisung von Luft gewonnen werden und es ist keine eigenständige Luftzerlegung notwendig, um Sauerstoff und/oder Stickstoff zu gewinnen. Dies ermöglicht weiterhin die Nutzung von Luft als Oxidationsmittel, so dass kein Umgang mit reinem Sauerstoff erforderlich ist. Besondere Vorteile ergeben sich ferner aus der Kombination von Stickstoff/Inertmedium und Dampf als Verdünnungsmittel.

Gemäß einer Variante des erfindungsgemäßen Verfahrens ist vorgesehen, dass sich der Einsatzstoffstrom (bei isothermer oder adiabatischer Reaktionsführung) zusammensetzt aus 1 Vol.-% bis 10 Vol.% Ethan, 1 Vol.% bis 5 Vol.-% O₂, 10 Vol.% bis 90 Vol.% H₂O, wobei der Rest N₂ ist, bevorzugt 1 Vol.-% bis 5 Vol.-% Ethan, 1 Vol.-% bis 3 Vol.-% O₂, 20 Vol.-% bis 90 Vol.-% H₂O, wobei der Rest N₂ ist, besonders bevorzugt 1 Vol.-% bis 3 Vol.-% Ethan, 1 Vol.-% bis 3 Vol.% O₂, 25 Vol.-% bis 90 Vol.-% H₂O, wobei der Rest N₂ ist, sowie - im Falle einer adiabatischer Reaktionsführung - besonders bevorzugt 2 Vol.-% bis 4 Vol.-% Ethan, 1 Vol.-% bis 3 Vol.-% O₂, 25 Vol.-% bis 90 Vol.-% H₂O, wobei der Rest N₂ ist.

Des Weiteren wird bei isothermer Reaktionsführung pro kg Katalysator vorzugsweise 1,0 kg bis 40 kg C₂H₆, bevorzugt 2 kg bis 25 kg C₂H₆, besonders bevorzugt 5 kg bis 20 kg C₂H₆ pro Stunde durch das mindestens eine Festbett geleitet. Bei adiabatischer Reaktionsführung wird bevorzugt pro kg Katalysator 1,0 kg bis 50 kg C₂H₆, bevorzugt 5 kg bis 30 kg C₂H₆, besonders bevorzugt 10 kg bis 25 kg C₂H₆ pro Stunde durch das mindestens eine Festbett geleitet.

Weiterhin wird das erfindungsgemäße Problem durch eine Reaktoreinrichtung mit den Merkmalen des Anspruchs 14 gelöst.

Danach weist die erfindungsgemäße Reaktoreinrichtung zumindest ein Festbett auf, das aus zumindest einer Mehrzahl an einen Katalysator aufweisenden ersten Partikeln gebildet ist, wobei erfindungsgemäß der Katalysator zur Beherrschung der Wärmetönung und/oder zur Vermeidung einer Explosion bei der oxidativen Dehydrierung mit einem Inertmaterial verdünnt ist.

Die Verdünnung des Katalysators ermöglicht - ggf. neben einer Kühlung der Reaktoreinrichtung mittels eines Kühlmittels (siehe unten) - mit Vorteil eine gute Beherrschung der Reaktionswärmeabfuhr und damit eine gleichmäßig hohe Reaktionstemperatur, was zu einem sicherem Betrieb führt. Die Reaktionswärme kann dabei zur Erzeugung von Hochdruck-Dampf als Nebenprodukt genutzt werden. Durch die gezielte Reaktionsführung kann ein fast vollständiger Alkan- bzw. Ethanumsatz erzielt werden, wobei man dann den O₂-Gehalt so bemessen kann, dass er am Reaktoraustritt minimal wird, also insbesondere außerhalb des Explosionsbereichs von Ethan/Ethylen-O₂ liegt.

Durch die gezielte Reaktionsführung, insbesondere die Verdünnung des Katalysatorfestbettes und die kurze Verweilzeit in einem ersten Festbett der Reaktoreinrichtung, wird im adiabatischen Fall des Weiteren ein Betrieb mit höherem Ethangehalt als im Vergleich zum Isothermreaktor im Feed erreichbar. Das Produktgas wird durch die Zwischenkühlung dabei soweit abgekühlt, dass es bei fast vollständigem Ethanumsatz in einem nachfolgenden zweiten Festbett auf eine Temperatur erhöht wird, die zu keinem Durchgehen der Reaktion führt. Der O₂-Gehalt zum zweiten Festbett ist dann nur noch so groß, dass er nicht zu einem Durchgehen der Reaktion führt. Zudem kann zur Zwischenkühlung auch noch Wasser eingespritzt werden, um die Kühlung effektiver zu machen und das Gasgemisch zu inhibieren (Wasserdampf erniedrigt Explosionsgrenzen). Das Produktgas aus dem zweiten Festbett enthält nur noch so viel O₂, dass eine Explosion nicht ermöglicht wird. Ferner kann die Zwischenkühlung nach dem ersten Festbett mit Vorteil zur Dampferzeugung genutzt werden.

In einer Variante der erfindungsgemäßen Reaktoreinrichtung ist weiterhin vorgesehen, dass jene ersten Partikel auch das Inertmaterial aufweisen (die ersten Partikel können z.B. als Pellets, als Extrudat oder in einer sonstigen Weise hergestellt sein).

Alternativ oder ergänzend hierzu ist bevorzugt vorgesehen, dass das mindestens eine Festbett zum Verdünnen des Katalysators eine Mehrzahl an mit den ersten Partikeln vermischten zweiten Partikeln aufweist, die aus dem Inertmaterial gebildet sind, wobei jene zweiten Partikel vorzugsweise regellos mit den ersten Partikeln vermischt sind. Die ersten und zweiten Partikel sowie das Inertmaterial können des Weiteren die vorstehend bereits beschriebenen Eigenschaften aufweisen.

In einer Ausführungsform der erfindungsgemäßen Reaktoreinrichtung ist vorgesehen, dass diese zur isothermen Reaktionsführung ausgebildet ist und zumindest einen Reaktor mit einem Reaktormantel aufweist, der einen Mantelraum zur Aufnahme eines Kühlmediums umschließt, sowie zumindest ein im Mantelraum angeordnetes Reaktorrohr, das das mindestens einen Festbett aufnimmt, so dass ein im Mantelraum befindliches Kühlmedium das mindestens eine Reaktorrohr zum Kühlen des mindestens einen Reaktorrohres umgibt. Vorzugsweise weist der Reaktor eine Mehrzahl an im Mantelraum angeordneten Reaktorrohren auf, die jeweils ein Festbett der vorgenannten Art aufnehmen. Bevorzugt ist weiterhin der mindestens eine Reaktor dazu ausgebildet, eine Flüssigkeit, ein Wärmeträgeröl oder eine Salzschmelze als Kühlmedium aufzunehmen. Bei der Flüssigkeit kann es sich z.B. im Wasser (insbesondere in Form von siedendem Wasser), einen Kohlenwasserstoff, z.B. in Form eines funktionalisierten Kohlenwasserstoffes (z.B. Alkohol) oder eines nichtfunktionalisierten Kohlenwasserstoffes, handeln.

Bei isothermer Reaktionsführung ist vorgesehen, dass der Anteil des Inertmaterials an dem mindestens einen Festbett bevorzugt 30 Vol.-% bis 90Vol.-%, bevorzugt 50Vol.-% bis 80Vol.-%, besonders bevorzugt 60Vol.% bis 80Vol.-% beträgt (der Rest wird durch den aktiven Katalysator gebildet).

In einer alternativen Variante der erfindungsgemäßen Reaktoreinrichtung ist vorgesehen, dass diese zur adiabatischen Reaktionsführung ausgebildet ist und stromab des mindestens einen Festbetts eine Einrichtung zum Zwischenkühlen eines aus dem mindestens einen Festbett austretenden Gasstroms (z.B. nicht umgesetzte Einsatzgase des Einsatzstoffstromes bzw. bei der oxidativen Hydrierung entstehender Produktstoffstrom) aufweist. Eine solche Einrichtung kann einen Wärmeübertrager zum Kühlen der besagten Stoffströme aufweisen und/oder kann dann dazu eingerichtet sein jene Stoffströme mit Wasser zu beaufschlagen.

Weiterhin weist die Reaktoreinrichtung stromab des mindestens einen Festbetts vorzugsweise einen Einlass zum Einspeisen von Einsatzgasen, insbesondere in Form von Sauerstoff, Luft und/oder Stickstoff auf (sogenannte Zwischeneinspeisung).

Vorzugsweise weist die adiabatische Reaktoreinrichtung stromab des mindestens einen Festbetts mehrere weitere hintereinander geschaltete Festbetten auf, wobei die Reaktoreinrichtung bevorzugt jeweils eine Einrichtung zum Kühlen bzw. Zwischenkühlen eines aus dem jeweiligen Festbett austretenden Stoffstroms (z.B. Einsatzgase bzw. bei der oxidativen Hydrierung entstehendes Produktgas) aufweist, bevor dieser in das nachfolgende Festbett eintritt.

Vorzugsweise weist die adiabatische Reaktoreinrichtung zwischen zwei aufeinanderfolgenden weiteren Festbetten jeweils einen Einlass zum Einspeisen von Einsatzgasen, insbesondere in Form von Sauerstoff, Luft und/oder Stickstoff auf.

Weiterhin kann bei einer adiabatischen Reaktoreinrichtung mit mehreren Festbetten der Katalysator des zweiten Festbettes bzw. der nachfolgenden Festbetten weniger verdünnt sein als das erste Festbett oder gar nicht verdünnt sein.
Bei einer adiabatischen Reaktoreinrichtung können des Weiteren die einzelnen Festbetten jeweils in separaten Reaktoren bzw. Reaktormänteln angeordnet sein.

Schließlich ist bei adiabatischer Reaktionsführung vorzugsweise vorgesehen, dass der Anteil des Inertmaterials an dem mindestens einen Festbett bevorzugt 30 Vol.-% bis 90 Vol.-%, bevorzugt 50 Vol.-% bis 85 Vol.-%, besonders bevorzugt 60 Vol.-% bis 80 Vol.-% beträgt.

Gemäß einer weiteren Variante der erfindungsgemäßen Reaktoreinrichtung kann diese Grundsätzlich einen oder mehrere separate Reaktoren aufweisen, wobei die Reaktoren zur isothermen oder zur adiabatischen Reaktionsführung eingerichtet und vorgesehen sein können (siehe oben). Solche Reaktoren können auch unterschiedlich gefahren werden. So kann ein erster Reaktor isotherm gefahren werden und ein nachfolgender weiterer Reaktor adiabatisch oder anders herum. Bevorzugt können weitere Reaktoren je einen Einlass zum zusätzlichen Einspeisen von Einsatzgasen wie z.B. Alkan (insbesondere Ethan) und/oder Sauerstoff aufweisen, wobei des Weiteren eine Einrichtung zum Zwischenkühlen eines aus einem vorhergehenden Reaktor austretenden Stoffstroms (z.B. Einsatzgase bzw. bei der oxidativen Hydrierung entstehendes Produktgas) vorgesehen sein kann, so dass der entsprechend gekühlte Stoffstrom in den jeweiligen weiteren Reaktor eingeleitet werden kann.

Insbesondere kann der Betrieb bei mehreren Reaktoren bei gleichen oder unterschiedlichen Bedingungen (Temperatur, Druck, insbesondere Partialdrücke der einzelnen Einsatzgase wie z.B. Sauerstoff) erfolgen.

Die vorstehend beschriebenen bzw. beanspruchten Reaktoreinrichtungen sind natürlich auch für andere oxidative Verfahrensweisen anwendbar. Bevorzugt ist jedoch der Anwendungsfall der oxidativen Dehydrierung von niederen Alkanen, insbesondere von Ethan und ggf. Propan und/oder Butan.

Weitere Einzelheiten und Vorteile der Erfindung sollen durch die nachfolgenden Figurenbeschreibungen von Ausführungsbeispielen anhand der Figuren erläutert werden.

Es zeigen:
- Fig. 1: eine schematische Schnittdarstellung einer erfindungsgemäßen, isotherm betriebenen Reaktoreinrichtung;
- Fig. 2: eine schematische Schnittdarstellung einer weiteren, adiabatisch betriebenen Reaktoreinrichtung;
- Fig. 3: ein Blockdiagramm eines erfindungsgemäßen Verfahrens zur oxidativen Dehydrierung von Alkanen (z.B. Ethan), bei dem eine Verdünnung des Einsatzstoffstromes mit Wasserdampf erfolgt;
- Fig. 4: ein Blockdiagramm eines weiteren erfindungsgemäßen Verfahrens zur oxidativen Dehydrierung von Alkanen (z.B. Ethan), bei dem eine Verdünnung des Einsatzstoffstromes mit Stickstoff erfolgt; und
- Fig. 5: ein Blockdiagramm eines weiteren erfindungsgemäßen Verfahrens zur oxidativen Dehydrierung von Alkanen (z.B. Ethan), bei dem eine Verdünnung des Einsatzstoffstromes mit Wasserdampf und Stickstoff erfolgt.

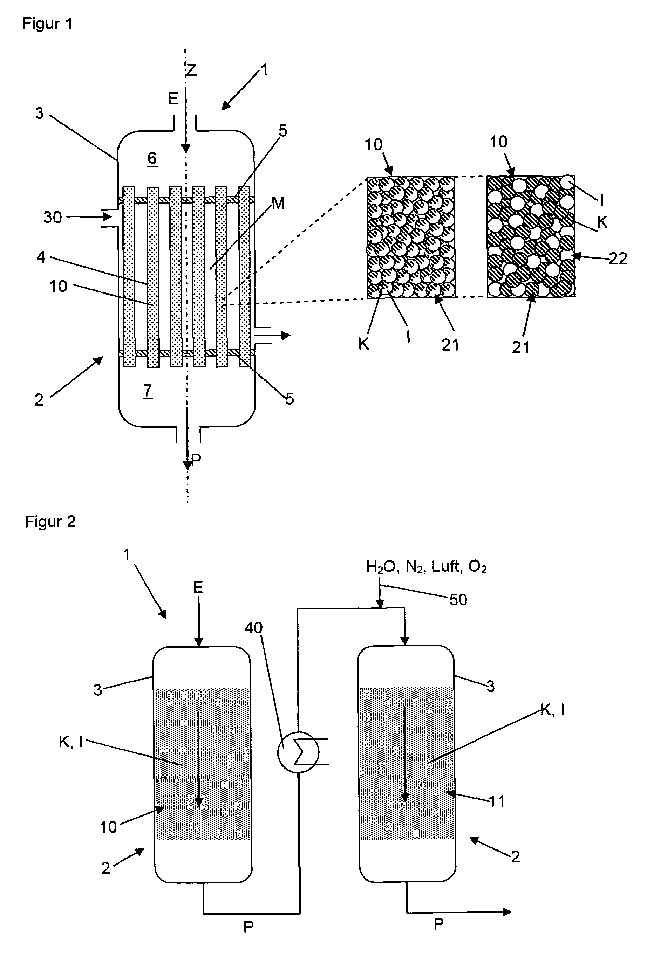

Figur 1 zeigt eine erfindungsgemäße Reaktoreinrichtung 1 mit zumindest einem Reaktor 2 zur isothermen Reaktionsführung. Der Reaktor 2 weist einen Reaktormantel 3 auf, der einen Mantelraum M zur Aufnahme eines Kühlmediums 30 in Form einer Flüssigkeit oder eines sonstigen geeigneten Kühlmediums 30 umschließt, sowie eine Mehrzahl an im Mantelraum M angeordneten Reaktorrohren 4, die jeweils ein Festbett 10 aufnehmen, das einen Katalysator K für die oxidative Dehydrierung von Ethan zu Ethylen aufweist. Die Reaktorrohre 4 sind von dem Kühlmedium 30 umgeben, so dass die Reaktionswärme der oxidativen Dehydrierung zur isothermen Reaktionsführung vom Kühlmedium 30 abgeführt werden kann. Die Reaktorrohre 4 sind dabei in Rohrböden 5 verankert, die vom Mantelraum M eine Einlass- sowie eine Auslasskammer 6, 7 abtrennen, wobei über die Einlasskammer 6 ein ethan- und sauerstoffhaltiger Einsatzstoffstrom E durch die Festbetten 10 entlang der Vertikale Z von oben nach unten durchgeleitet wird. In den einzelnen Festbetten 10 entsteht dabei durch oxidatives Dehydrieren des Ethans ein ethylenhaltiger Produktstoffstrom P, der über die Auslasskammer 7 aus dem Reaktor 2 abgezogen wird.

Wie in Figur 1 (Detail mitte) gezeigt, besteht in einer ersten Variante das jeweilige Festbett 10 aus zumindest einer Mehrzahl an den Katalysator K aufweisenden ersten Partikeln 21, die zum Verdünnen des Katalysators K des Weiteren ein Inertmaterial I aufweisen, bei dem es sich z.B. um Aluminiumoxid, Siliciumdioxid, Siliciumcarbid, Quartz oder Keramik handeln kann.

Gemäß einer weiteren Variante (Detail Figur 1 rechts) ist vorgesehen, dass das jeweilige Festbett 10 zum Verdünnen des Katalysators K eine Mehrzahl an mit den ersten Partikeln 21 regellos vermischten zweiten Partikeln 22 aufweist, die aus dem Inertmaterial I gebildet sind. Hierbei sind die Partikel 21, 22 so vermischt, dass in dem jeweiligen Festbett 10 pro Volumeneinheit eine konstante Dichte an ersten bzw. zweiten Partikeln 21, 22 herrscht.

Durch die Verdünnung des Katalysators K mit Inertmaterial I entsteht pro Volumeneinheit in den Reaktorrohren 4 weniger Wärme, die durch das Kühlmedium 30 auf der Außenseite leichter abgeführt werden kann. Der Grad der Verdünnung wird so bemessen, dass die Reaktionstemperatur in den Reaktorrohren 4 so hoch gehalten werden kann, dass größtmögliche Umsätze und Selektivitäten erreicht werden, ohne dass es zu einem Durchgehen der Reaktion führt. Das Inertmaterial I bzw. die zweiten Partikel 22 können dabei auch eine andere Form oder eine andere Größe aufweisen als der aktive Katalysator K bzw. die ersten Partikel 21, um dadurch den Druckabfall beim Durchströmen des Festbetts 10 bzw. der Schüttung 10 klein zu halten.

### Beispiel 1

Prozessdaten für die Reaktoreinrichtung 1 in Form eines Isothermreaktors gemäß Figur 1:
- Druck im Reaktor 2: von 0,5 bar bis 35 bar, bevorzugt 1 bar bis 15 bar, besonders bevorzugt 1 bar bis 5 bar.
- Temperatur im Reaktor 2: zwischen 250°C bis 650°C, bevorzugt 280°C bis 550°C, besonders bevorzugt 350°C bis 480°C.
- Feedzusammensetzungen (Einsatzstoffstrom E): 1 Vol.-% bis 10 Vol.-% Ethan, 1 Vol.-% bis 5 Vol.-% O₂, 10 Vol.-% bis 95 Vol.-% H₂O, Rest N₂, bevorzugt 1 Vol.-% bis 5 Vol.-%, 1 Vol.-% bis 3 Vol.-% O₂, 20 Vol.-% bis 90 Vol.-% H₂O, Rest N₂, besonders bevorzugt 1 Vol.-% bis 3 Vol.-% Ethan, 1 Vol.-% bis 3 Vol.-% O₂, 25 Vol.-% bis 90 Vol.-% H₂O, Rest N₂.
- WHSV = 1.0 kg bis 40 kg C₂H₆/h/kgKat, bevorzugt WHSV = 2kg bis 25 kg C₂H₆/h/kgKat, besonders bevorzugt WHSV = 5 kg bis 20 kg C₂H₆/h/kgKat.
- Der Anteil des Inertmaterials I an den Festbetten 10 beträgt 30 Vol.-% bis 90 Vol.-%, bevorzugt 50 Vol.-% bis 80 Vol.-%, besonders bevorzugt 60 Vol.-% bis 75 Vol.-%.

Gemäß Figur 2 kann die erfindungsgemäße Reaktoreinrichtung 1 auch adiabatisch betrieben werden. Hierbei sind bevorzugt mehrere Festbetten 10, 11 der oben beschriebenen Art (insbesondere Verdünnung mit Inertmaterial I) hintereinander geschaltet, so dass der Einsatzstoffstrom E bzw. Produktstoffstrom P diese nacheinander durchlaufen kann. Hierbei wird zwischen zwei Festbetten 10, 11 eine Zwischenkühlung des aus dem jeweiligen vorangegangenen Festbett 10 ausgetretenen Stoffstromes P vorgenommen, wobei des Weiteren über einen Einlass 50 stromauf der nächsten Stufe bzw. stromauf des nächsten Festbettes 11 eine Zwischeneinspeisung von Luft oder Sauerstoff vorgenommen wird. In der Figur 2 sind exemplarisch lediglich zwei Festbetten 10, 11 gezeigt. Es können natürlich auch mehr als zwei Festbetten 10, 11 vorhanden sein. Die Festbetten 10, 11 können dabei in separaten Reaktoren 2 bzw. Reaktormänteln 3 der Reaktoreinrichtung 1 angeordnet sein.

Der Grad der Verdünnung des aktiven Katalysators K und die Länge des ersten Festbettes 10 sowie der Gehalt an Ethan im Einsatzstoffstrom E bzw. Gaszustrom E zum ersten Festbett 10 wird dabei so bemessen, dass der adiabatische Temperaturanstieg höchste Umsätze bei höchster Selektivität ermöglicht. In dem nachgeschalteten zweiten Festbett 11 oder einem weiteren Festbett kann auf eine Verdünnung des aktiven Katalysators K verzichtet werden, um den vollständigen Umsatz von Ethan zu ermöglichen.

### Beispiel 2

Prozessdaten für adiabatische Reaktoreinrichtung 1 gemäß Figur 2 mit Zwischenkühlern 40 vor dem nächsten Festbett 11 :
- Druck in Reaktoren 2: von 0,5 bar bis 35 bar, bevorzugt 1 bar bis 15 bar, besonders bevorzugt 5 bar bis 15 bar.
- Temperatur in Reaktoren 2: zwischen 250°C bis 650°C, bevorzugt 280°C bis 550°C, besonders bevorzugt 350°C bis 480°C.
- Feedzusammensetzungen (Einsatzstoffstrom E): 1 Vol.-% bis 10 Vol.-% Ethan, 1 Vol.-% bis 5 Vol.-% O₂, 10 Vol.-% bis 95 Vol.-% H₂O, Rest N₂, bevorzugt 1 Vol.-% bis 5 Vol.-% Ethan, 1 Vol.-% bis 3 Vol.-% O₂, 20 Vol.-% bis 90 Vol.-% H₂O, Rest N₂, besonders bevorzugt 2 Vol.-% bis 4 Vol.-% Ethan, 1 Vol.-% bis 3 Vol.-% O₂, 25 Vol.-% bis 90 Vol.-% H₂O, Rest N₂
- WHSV = 2 kg bis 50 kg C₂H₆/h/kgKat, bevorzugt WHSV = 5 kg bis 30 kg C₂H₆/h/kgKat, besonders bevorzugt WHSV = 10 kg bis 25 kg C₂H₆/h/kgKat.
- Der Anteil des Intermaterials I an den Festbetten 10, 11 beträgt 30 Vol.-% bis 90 Vol.-%, bevorzugt 50 Vol.-% bis 85 Vol.-%, besonders bevorzugt 60 Vol.-% bis 80 Vol.-%. Das zweite oder ein weiteres Festbett 11 kann ohne Intertmaterial I ausgeführt sein.

Für beide Reaktionsführungen (isotherm und adiabatisch) kann das Inertmaterial I auch in den Katalysator K eingearbeitet sein (siehe oben). Die entsprechenden Partikel bzw. Formkörper 21 (Pellets, Extrudat oder andere) können also aus einer Mischung aus Inert- und Aktivmaterial K erzeugt werden.

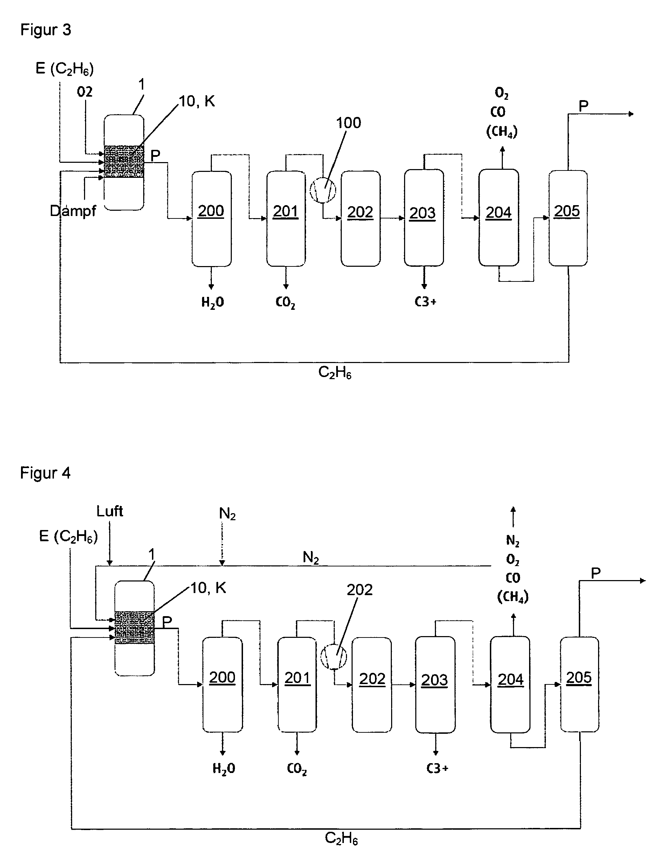

Figur 3 zeigt eine weitere Variante des erfindungsgemäßen Verfahrens zur oxidativen Dehydrierung von Alkanen, hier in Form von Ethan.

Danach werden als Einsatzgase (Einsatzstoffstrom E) ein Alkan, vorliegend Ethan, sowie Sauerstoff als Oxidationsmittel in einer Reaktoreinrichtung 1 einem Katalysator K zugeführt, bei dem es sich bevorzugt um einen MoVTeNbOₓ-Katalysator handelt, wobei das Ethan oxidativ unter Entstehung eines ethylenhaltigen Produktstoffstromes P in Gegenwart des Katalysators K dehydriert wird (anstelle von Ethan kommen als Einsatz insbesondere auch Propan und/oder Butan in Frage), wobei zur kontrollierten Reaktion außerhalb von Explosionsbereichen ein inertes Verdünnungsmittel in die Reaktoreinrichtung 1 in Form von Wasserdampf eingeleitet wird. Als Reaktoreinrichtung 1 kann prinzipiell eine Reaktoreinrichtung 1 gemäß Figur 1 oder 2 verwendet werden. Weiterhin kann der nachfolgend beschriebene Zerlegungsprozess auch dem hinsichtlich der Figuren 1 und 2 beschriebenen Verfahren nachgeschaltet werden.

Der ethylenhaltige Stoffstrom P wird nun aus der Reaktoreinrichtung 1 abgezogen und in einer entsprechenden Kolonne einer Wasserwäsche 200 zugeführt, die als erste Reinigungsstufe (insbesondere Auswaschen wasserlöslicher Bestandteile wie z.B. Carbonsäure, insbesondere Essigsäure, und/oder Carbonylverbindungen, wie z.B. Aldehyde oder Ketone) des Produktstoffstromes P dient, wobei das auskondensierte Wasser aus dem Sumpf der Kolonne 200 abgezogen und als Makeupwasser der Wäsche genutzt wird.

Der solchermaßen vorgereinigte Produktstoffstrom P wird sodann insbesondere zum Entfernen von bei der oxidativen Dehydrierung entstandenem CO₂ einer Amin- und/oder Laugewäsche 201 unterzogen, wonach der Produktstoffstrom P in einem Verdichter 100 verdichtet und in einer Adsorberstation 202 getrocknet wird.

Hiernach wird der Produktstoffstrom P in eine Deethanisierer-Kolonne 203 (auch als Deethanizer bezeichnet) eingespeist, wobei ein C₂₋-Strom enthaltend u.a. Ethan und Ethylen über Kopf abgezogen wird und einer Demethanisierer-Kolonne (auch als Demethanizer bezeichnet) 204 zugeführt wird. Der C₃₊-Strom wird aus dem Sumpf des Deethanizers 203 abgezogen.

Aus dem Demethanizer 204 werden dann über Kopf die Komponenten O₂, CO und CH₄ abgezogen, wobei der entstehende ethan- und ethylenhaltige Produktstoffstrom P in einen C2-Splitter 205 gegeben wird, aus dem über Kopf das Ethylenprodukt P abgezogen wird. Das aus dem Sumpf des C2-Splitters abgezogene Ethan wird in die Reaktoreinrichtung 1 als Einsatz zurückgeführt.

In einer in Figur 4 gezeigten Abwandlung des Verfahrens gemäß Figur 3 wird anstelle von Sauerstoff Luft als Verdünnungs- bzw. Oxidationsmittel in die Reaktoreinrichtung 1 eingeleitet. Dabei kann Stickstoff und/oder Sauerstoff in der Luft an- oder abgereichert sein. Durch Reaktion des in der Luft enthaltenen Sauerstoffes verbleiben Stickstoff und inerte Komponenten aus der Luft im Produktstrom P. Der Stickstoff und Inerte werden so dann im Zerlegungsteil 204 abgetrennt und als Verdünnungsmittel in die Reaktoreinrichtung 1 zurückgeführt. Lediglich bei Inbetriebnahme (Startup) ist ggf. die Zuführung eines Stickstoff-Makeupstromes erforderlich. Angesammelte Verunreinigungen können über einen Purge-Strom ausgeschleust werden. Je nach Erfordernis kann eine zusätzliche Einspeisung von reinem N₂ erfolgen, um die Sauerstoffkonzentration gering zu halten und/oder hohe Purgeraten zu ermöglichen.

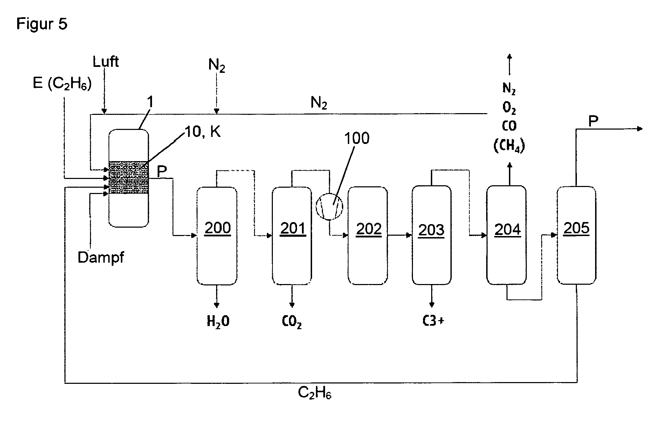

Weiterhin zeigt Figur 5 eine Kombination der Verfahren gemäß Figuren 3 und 4. Danach wird zusätzlich zum Stickstoff Wasserdampf als Verdünnungsmittel in die Reaktoreinrichtung 1 eingeleitet. Auf diese Weise können die Vorteile der Varianten gemäß Figuren 3 und 4 kombiniert werden.

Grundsätzlich kann beim Anfahren der Anlage Wasserdampf oder Stickstoff oder ein Gemisch dieser beiden Stoffe in die Reaktoreinrichtung 1 eingeleitet werden. Sodann werden die Reaktanden für die oxidative Dehydrierung in die Reaktoreinrichtung 1 gegeben, also das verwendete Alkan sowie das verwendete Oxidationsmittel, wobei das Oxidationsmittel in Form von Sauerstoff oder Luft hinzugegeben werden kann. In der Luft kann dabei Stickstoff und/oder Sauerstoff jeweils an- oder abgereichert sein. In die Reaktoreinrichtung 1 gegebener Stickstoff wird, wie oben beschrieben, nach Abtrennung bevorzugt in die Reaktoreinrichtung 1 zurückgeführt.

Nach dem Anfahren kann das beim Anfahren hinzugegebene Verdünnungsmittel weiterhin in die Reaktoreinrichtung 1 eingeleitet werden oder zumindest teilweise oder auch vollständig durch ein anderes der genannten Verdünnungsmittel ersetzt werden. Ein anderes der genannten Verdünnungsmittel kann auch zusätzlich in die Reaktoreinrichtung 1 eingeleitet werden.

### Beispiel 3

Prozessdaten für das Verfahren zur oxidativen Dehydrierung gemäß Figuren 3 bis 5:
- Druck in Reaktoreinrichtung 1: von 0,5 bar bis 35 bar, bevorzugt 1 bar bis 15 bar, besonders bevorzugt 1 bar bis 5 bar.
- Temperatur in Reaktoreinrichtung 1: 250°C bis 650°C, bevorzugt 280°C bis 550°C, besonders bevorzugt 350°C bis 480°C.
- Feed (Einsatzstoffstrom E): n-Alkane, bevorzugt C2-C6-Alkane, besonders bevorzugt Ethan.
- Zuführung eines Verdünnungsmittels bzw. Intertmediums (Wasserdampf, Stickstoff, Edelgase, andere unter Reaktionsbedingungen inerte Gase, sowie beliebige Mischungen davon), bevorzugt sind Wasser und Stickstoff bzw. deren Mischungen.
- Verwendung von reinem Sauerstoff oder Luft als Oxidationsmittel.

Die Verfahrensvarianten gemäß Figuren 3 bis 5 können mit einem beliebigen Katalysator durchgeführt werden, der unter den Reaktionsbedingungen stabil ist. Bevorzugt wird vorliegend jedoch ein MoVTeNbOₓ-Katalysator verwendet.

### Bezugszeichenliste

| | |
|---|---|
| 1 | Reaktoreinrichtung |
| 2 | Reaktor |
| 3 | Reaktormantel bzw. Reaktorrohr |
| 4 | Reaktorrohr |
| 5 | Rohrboden |
| 6 | Einlasskammer |
| 7 | Auslasskammer |
| 10, 11 | Festbett |
| 21 | Erste Partikel (Schüttfüllkörper) |
| 22 | Zweite Partikel (Schüttfüllkörper) |
| 30 | Kühlmedium (z.B.siedendes Wasser) |
| 40 | Einrichtung zum Kühlen |
| 50 | Einlass (Zwischeneinspeisung) |
| 100 | Verdichter |
| 200 | Wasserwäsche(-Kolonne) |
| 201 | Waschkolonne (Amin- und/oder Laugenwäsche) |
| 202 | Trocknung |
| 203 | Deethanisierer |
| 204 | Demethanisierer |
| 205 | C2-Splitter |
| E | Einsatzstoffstrom |
| I | Inertmaterial |
| K | (aktiver) Katalysator |
| M | Mantelraum |
| P | Produktstoffstrom |
| Z | Vertikale |

## Patentansprüche

1. Verfahren zur oxidativen Dehydrierung von Alkanen, insbesondere von Ethan zu Ethylen, bei dem ein Einsatzstoffstrom (E) enthaltend ein Alkan, insbesondere Ethan, sowie Sauerstoff durch zumindest ein einen Katalysator (K) enthaltendes Festbett (10, 11) einer Reaktoreinrichtung (1) geleitet wird, wobei durch oxidatives Dehydrieren des Alkans mit Sauerstoff in Gegenwart des Katalysators (K) ein alkenhaltiger Produktstoffstrom (P) erzeugt wird, und wobei der Katalysator (K) mit einem Inertmaterial (I) verdünnt ist.

2. Verfahren nach Anspruch 1, **dadurch gekennzeichnet, dass** das mindestens eine Festbett (10, 11) aus zumindest einer Mehrzahl an jenen Katalysator (K) aufweisenden ersten Partikeln (21) gebildet ist, wobei insbesondere jene ersten Partikel (21) auch das Inertmaterial (I) aufweisen und/oder wobei das mindestens eine Festbett (10, 11) zum Verdünnen des Katalysators (K) eine Mehrzahl an mit den ersten Partikeln (21) vermischte zweite Partikel (22) aufweist, die aus dem Inertmaterial (I) gebildet sind.

3. Verfahren nach Anspruch 1 oder 2, **dadurch gekennzeichnet, dass** die oxidative Dehydrierung isotherm erfolgt, wobei das mindestens eine Festbett (10) mittels einer Flüssigkeit (30), eines Wärmeträgeröls oder einer Salzschmelze gekühlt wird, und/oder dass die oxidative Dehydrierung adiabatisch erfolgt, wobei insbesondere der Einsatzstoffstrom (E) durch eine Mehrzahl an aufeinanderfolgenden Festbetten (10, 11) gegeben wird, wobei insbesondere der Einsatzstoffstrom (E) zwischen je zwei benachbarten Festbetten (10, 11) zwischengekühlt wird, insbesondere durch Einspritzen von Wasser, und/oder dem Einsatzstoffstrom (E) Sauerstoff, Stickstoff und/oder Luft hinzugegeben wird, wobei insbesondere in der Luft Sauerstoff und/oder Stickstoff an- oder abgereichert ist.

4. Verfahren nach einem der vorhergehenden Ansprüche, **dadurch gekennzeichnet, dass** der Anteil des Inertmaterials (I) an dem mindestens einen Festbett (10) bei isothermer Reaktionsführung 30 Vol.-% bis 90 Vol.-%, bevorzugt 50 Vol.-% bis 80 Vol.-%, besonders bevorzugt 60 Vol.% bis 75 Vol.-% beträgt, oder dass der Anteil des Inertmaterials (I) an dem mindestens einen Festbett (10, 11) bei adiabatischer Reaktionsführung 30 Vol.-% bis 90 Vol.-%, bevorzugt 50 Vol.-% bis 85 Vol.-%, besonders bevorzugt 60 Vol.% bis 80 Vol.-% beträgt.

5. Verfahren nach einem der vorhergehenden Ansprüche, **dadurch gekennzeichnet, dass** der Einsatzstoffstrom (E) bei einem Druck von 0,5 bar bis 35 bar, bevorzugt 1 bar bis 15 bar, besonders bevorzugt 1 bar bis 10 bar durch das mindestens eine Festbett (10, 11) geleitet wird.

6. Verfahren nach einem der vorhergehenden Ansprüche, **dadurch gekennzeichnet, dass** der Einsatzstoffstrom (E) bei einer Temperatur von 250°C bis 650°C, bevorzugt 280°C bis 550°C, besonders bevorzugt 350°C bis 480°C durch das mindestens eine Festbett (10, 11) geleitet wird.

7. Verfahren nach einem der vorhergehenden Ansprüchen, **dadurch gekennzeichnet, dass** der Einsatzstoffstrom (E) mit einem Verdünnungsmittel verdünnt wird, wobei das Verdünnungsmittel inert ist oder zumindest eine inerte Komponente aufweist.

8. Verfahren nach Anspruch 7, **dadurch gekennzeichnet, dass** als Verdünnungsmittel Wasserdampf, Stickstoff, und/oder Luft verwendet wird, wobei insbesondere Stickstoff und/oder Sauerstoff in der besagten Luft an- oder abgereichert ist.

9. Verfahren nach Anspruch 7 oder 8, **dadurch gekennzeichnet, dass** beim Anfahren Wasserdampf und/oder Stickstoff als Verdünnungsmittel in die Reaktoreinrichtung (1) gegeben wird und sodann besagtes Alkan und als Oxidationsmittel der Sauerstoff oder den Sauerstoff enthaltene Luft hinzugegeben werden, wobei insbesondere in der Luft Sauerstoff und/oder Stickstoff an- oder abgereichert ist, und wobei insbesondere das beim Anfahren hinzugegebene Verdünnungsmittel nach dem Anfahren weiterhin in die Reaktoreinrichtung (1) gegeben wird oder zumindest teilweise oder vollständig durch ein anderes Verdünnungsmittel ersetzt wird.

10. Verfahren nach einem der vorhergehenden Ansprüche, **dadurch gekennzeichnet, dass** sich der Einsatzstoffstrom (E) zusammensetzt aus 1 Vol.-% bis 10 Vol.-% Ethan, 1 Vol.-% bis 5 Vol.-% O₂, 10 Vol.-% bis 95 Vol.-% H₂O, Rest N₂, bevorzugt 1 Vol.-% bis 5 Vol.-% Ethan, 1 Vol.-% bis 3 Vol.-% O₂, 20 Vol.-% bis 90 Vol.-% H₂O, Rest N₂, besonders bevorzugt 1 Vol.-% bis 3 Vol.-% Ethan, 1 Vol.-% bis 3 Vol.-% O₂, 25 Vol.-% bis 90 Vol.-% H₂O, Rest N₂, sowie im Falle einer adiabatischen Reaktionsführung besonders bevorzugt 2 Vol.-% bis 4 Vol.-% Ethan, 1 Vol.-% bis 3 Vol.-% O₂, 25 Vol.-% bis 90 Vol.-% H₂O, Rest N₂.

11. Verfahren nach einem der vorhergehenden Ansprüche, **dadurch gekennzeichnet, dass** bei isothermer Reaktionsführung pro kg Katalysator 1,0 kg bis 40 kg C₂H₆, bevorzugt 2 kg bis 25 kg C₂H₆, besonders bevorzugt 5 kg bis 20 kg C₂H₆ pro Stunde durch das mindestens eine Festbett (10) geleitet wird, oder dass bei adiabatischer Reaktionsführung pro kg Katalysator 1,0 kg bis 50 kg C₂H₆, bevorzugt 5 kg bis 30 kg C₂H₆, besonders bevorzugt 10 kg bis 25 kg C₂H₆ pro Stunde durch das mindestens eine Festbett (10, 11) geleitet wird.

12. Verfahren nach Anspruch 2 oder einem der Ansprüche 3 bis 11, **dadurch gekennzeichnet, dass** die zweiten Partikel (22) eine von den ersten Partikeln (21) abweichende Geometrie oder Dimensionierung aufweisen.

13. Verfahren nach einem der vorhergehenden Ansprüche, **dadurch gekennzeichnet, dass** das Inertmaterial (I) einen oder mehrere der folgenden Stoffe aufweist: Aluminiumoxid, Siliciumdioxid, Siliciumcarbid, Quartz oder Keramik.

14. Reaktoreinrichtung zur oxidativen Dehydrierung von Alkanen, insbesondere zur Verwendung bei einem Verfahren gemäß einem der vorhergehenden Ansprüchen, mit:
- zumindest einem Festbett (10, 11), das aus zumindest einer Mehrzahl an einen Katalysator (K) aufweisenden ersten Partikeln (21) gebildet ist,
**dadurch gekennzeichnet,**
**dass** der Katalysator (K) mit einem Inertmaterial (I) verdünnt ist.

15. Reaktoreinrichtung nach Anspruch 14, **dadurch gekennzeichnet, dass** jene ersten Partikel (21) auch das Inertmaterial (I) aufweisen, und/oder dass das mindestens eine Festbett (10, 11) zum Verdünnen des Katalysators (K) eine Mehrzahl an mit den ersten Partikeln (21) vermischten zweiten Partikeln (22) aufweist, die aus dem Inertmaterial (I) gebildet sind.
